# EUROPEAN PATENT APPLICATION

(11) **EP 1 923 047 A1**
(43) Date of publication of application: **21.05.2008**
(21) Application number: 06797598.7
(22) Date of filing: 07.09.2006
(51) Int. Cl.: A61K 8/89, A61K 8/92, A61Q 1/02

(54) **POWDER-CONTAINING TRANSPARENT SOLID COSMETIC PREPARATION**

(30) Priority: 07.09.2005 JP 2005259963
(71) Applicant: Kao Corporation, Chuo-ku Tokyo 103-8210 (JP)
(72) Inventor: SHIMIZU, Kazuhiro, 3-28, Kotobuki-cho 5-chome, Odawara-shi, Kanagawa 250-0002 (JP)
(74) Representative: Green, Mark Charles
(86) International application number: PCT/JP2006/317722
(87) International publication number: WO 2007/029761

(57) **Abstract**

The present invention provides a novel solid powder which has transparency in its appearance, and has excellent usability and, in addition, excellent feeling of use such as dry feeling and fresh light feeling without sticky feeling during application. It is achieved by a solid powder cosmetic comprising: (A) 25 to 55% by mass of elastic powder mixture; (B) 20 to 40% by mass of non-elastic spherical silicone resin powder with an average particle diameter within the range of 0.1 to 50 µm; and (C) 25 to 55% by mass of oil, as essential components, wherein (A) the elastic powder mixture comprises one or more types of each (A1) an elastic powder and (A2) a composite powder obtained by coating the periphery of an elastic powder with a non-elastic material, said solid powder cosmetic being obtained by caking a mixed composition of these essential components.

## Description

### [Technical Field]

The present invention relates to a solid powder cosmetic which has an excellent aesthetic sense being transparent in its appearance, has excellent usability such as easiness to take when using a puff, a brush, or the like, and has improved feeling of use such as dry feeling, fresh light feeling, or the like.

### [Background Art]

In recent years, developments have been made for cosmetics with transparent appearance such that they have various formulations for the purpose of achieving visual beauty or the like as an added value. Among these transparent cosmetics, quite many considerations have been made with regard to those constituted mainly from aqueous components such as a lotion, an emulsion, or the like (see, e.g., Patent References 1 and 2) and an oily formulation in which 12-hydroxystearic acid is formulated (see, e.g., Patent Reference 3).

On the other hand, it is very difficult to apply the transparency technology to a powder cosmetic, in the respect that it has less oil content as a binder. In Patent Reference 4, the present applicant disclosed the invention of a solid powder cosmetic of a mixed composition of elastic powders, non-elastic spherical silicone resin powders and an oil which is transparent in its appearance and has excellent usability such as easiness to take. However, a development of a cosmetic having more excellent usability has been required.
[Patent Reference 1]
Japanese Patent Publication No. 2752231
[Patent Reference 2]
Japanese Laid-Open Patent [Kokai] Publication No. 2005-53834
[Patent Reference 3]
Japanese Laid-Open Patent [Kokai] Publication No. Hei 11-222413
[Patent Reference 4]
Japanese Patent Publication No. 3621233

### [Disclosure of the Invention]

### [Problems to be Solved by the Invention]

The present invention has been made taking account of the above-mentioned matters, and is aimed to provide a. novel solid powder cosmetic which has transparency in its appearance, and has excellent usability and, in addition, excellent feeling of use such as dry feeling and fresh light feeling without sticky feeling during application.

### [Means for Solving the Problems]

Taking into consideration such circumstances, the present inventors have conducted dedicated research in order to improve the feeling of use such as dry feeling and fresh light feeling in a solid powder cosmetic with transparent appearance, and as a result, have found that a transparent solid powder cosmetic having transparency in its appearance and being excellent in usability can be obtained by mixing certain ratio of multiple particular essential components with optional any components and caking these components, whereby the present invention has been completed.

Namely, the present invention direct to a solid powder cosmetic comprising : (A) 25 to 55% by mass of elastic powder mixture; (B) 20 to 40% by mass of non-elastic spherical silicone resin powder with an average particle diameter within the range of 0.1 to 50 µm; and (C) 25 to 55% by mass of oil, as essential components, wherein (A) the elastic powder mixture comprises one or more types of each (A1) an elastic powder and (A2) a composite powder obtained by coating the periphery of an elastic powder with a non-elastic material, said solid powder cosmetic being obtained by caking a mixed composition of these essential components and optional any components.
Furthermore, the present invention direct to a solid powder cosmetic comprising: (A) 30 to 55% by mass of elastic powder mixture; (B) 25 to 40% by mass of non-elastic spherical silicone resin powder with an average particle diameter within the range of 0.1 to 50 µm; and (C) 20 to 45% by mass of oil, as essential components, wherein (A) the elastic powder mixture comprises one or more types of each (A1) an elastic powder and (A2) a composite powder obtained by coating the periphery of an elastic powder with a non-elastic material, said solid powder cosmetic being obtained by caking a mixed composition of these essential components and optional any components.

### [Effect of the Invention]

The present invention allows to provide a transparent solid powder cosmetic which has an excellent aesthetic sense being transparent in its appearance, has excellent usability such as easiness to take when using a puff, a brush, or the like, and has improved feeling of use such as dry feeling, fresh light feeling, or the like.

### [Best Mode for Carrying Out the Invention]

In the following, embodiments of the present invention are described in detail.

(A) The elastic powder mixture used in the present invention comprises one or more types of each (A1) an elastic powder and (A2) a composite powder obtained by coating the periphery of an elastic powder with a non-elastic material.
(A1) The elastic powder is a powder having rubber elasticity, which is an aggregate of primary particles with an average particle diameter of 0.01 to 1000 µm, preferably 0.1 to 50 µm based on the observation using an electron microscope, having the JIS-A hardness measured according to JIS-K-6301 in the range of 15 to 80. Specific examples of such an elastic powder include a powder of synthetic elastomers such as silicone elastomer, fluorosilicone elastomer, modified silicone elastomer, acrylic elastomer and polyurethane elastomer, natural elastomers, and the like. Among these, a powder of silicone elastomer is most preferred. It does not matter if the powder of silicone elastomer contains a silicone oil such as dimethylpolysiloxane. Commercially available products include Torayfil E series from Toray Dow Corning Co., Ltd., such as Torayfil E-506C (100% of powder content), Torayfil E-507 (70% of powder content), Torayfil E-508 (70% of powder content), and the like.

(A2) The composite powder obtained by coating the periphery of a elastic powder with a non-elastic material refers to a powder having rubber elasticity which is an aggregate of primary particles, which is one obtained by coating the surface of a powder having the JIS-A hardness measured according to JIS-K-6301 in the range of 15 to 80 with the non-elastic material to composite them, and the one in which the hardness of the composite powder as a whole is within 15 to 80. The average particle size is 0.01 to 1000 µm, preferably 0.1 to 50 µm. Specific examples of such elastic powders can include the one same as the (A1) elastic powder. Further, the non-elastic material refers to a material of which hardness shows a numerical value greater (harder) than the hardness of the elastic powder to be coated. Specifically, the non-elastic material includes organic resins such as silicone resin, acrylic resin and nylon resin, and inorganic resins such as silica and alumina. Among these, silicone resin, in particular, polyorganosilsesquioxane hardened material is preferably used.

Specific examples of (A2) the composite powder obtained by coating the periphery of an elastic powder with a non-elastic material include a composite silicone powder obtained by coating the periphery of a silicone elastomer powder with a silicone resin, more specifically a composite powder obtained by coating the peripheral of a silicone elastomer powder with polyorganosilsesquioxane hardened material, etc. Such composite powders, as commercially available products, include KSP series from Shin-Etsu Chemical Co., Ltd., such as KSP-100, KSP-101, KSP-102, KSP-105, KSP-300 and the like. Also, such composite powders can be prepared according to the method described in Japanese Laid-Open Patent [Kokai] Publication No. 2000-38314 or Japanese Patent Publication No. 2832143.

For a combination of (A1) the elastic powder and (A2) the composite powder obtained by coating the periphery of an elastic powder with a non-elastic material, (A) an elastic powder mixture in which the component (A1) is silicone elastomer powder and the component (A2) is a composite silicone powder obtained by coating the periphery of silicone elastomer powder with silicone resin can be preferably used.

It is preferable that the blending ratio (weight ratio) of (A1) the elastic powder and (A2) the composite powder obtained by coating the periphery of an elastic powder with a non-elastic material included in (A) the elastic powder mixture is in the range of 2:1 to 20:1. Furthermore, it is preferable that the blending amount of (A2) the composite powder obtained by coating the periphery of an elastic powder with a non-elastic material is 3% or more by mass based on the total amount of the cosmetics, more preferable that it is 5% or more by mass. If the blending amount is within such range, fresh light feeling and dry feeling can be achieved for the feeling of use.

These (A) elastic powder mixtures comprise one or more types of each (A1) and (A2), whereby it is possible to obtain fresh light feeling and dry feeling without losing transparency.

The blending amount of these (A) elastic powder mixtures is 25 to 55% by mass based on the total amount of the cosmetics, preferably 25 to 45% by mass, more preferably 30 to 40% by mass. If the blending amount is 25% by mass or more, then the cosmetic has excellent transparency in its appearance, and if the blending amount is 55% by mass or less, then the cosmetic has good usability such as easiness to take.

Further, as (A) the elastic powder mixtures, an elastic powder mixture which comprises elastic powders obtained by treating the surface thereof with conventionally known compounds such as trimethylsiloxysilicate, a silane coupling agent, a metallic soap, N-acylated lysine and perfluoroalkylphosphate ester salt, or elastic powders obtained through mechano-chemical treatment, plasma treatment, or the like may also be applied. In the use of it, it does not matter if the treated elastic powders are used as they are, it is preferable to use them after releasing the aggregation using a bead mill, a triturating machine, or the like.

(B) A non-elastic spherical silicone resin powder used in the present invention refers to a powder of spherical silicone resin with an average particle diameter of 0.1 to 50 µm, preferably 1 to 20 µm based on the observation using an electron microscope, having the JIS-A hardness over 80, preferably that of 90 or more.
As (B) a non-elastic spherical silicone resin powder used in the present invention, there may be used a silicone resin powder such as methylsiloxane network polymer:or polyorganosilsesquioxane (polymethylsilsesquioxane) hardened material. Specific examples thereof include Tospearl series from GE Toshiba Silicone Co., Ltd.
Taking account of "spread" on a skin and "adhesiveness to skin" of the cosmetic, the average particle diameter of these (B) components are required to be 0.1 to 50 µm, preferably 1 to 20 µm.

The blending amount of (B) a non-elastic spherical silicone resin powder is 20 to 40% by mass based on the total amount of the cosmetics, preferably 25 to 40% by mass, more preferably 25 to 35% by mass. If the blending amount is within such range, not only dry feeling and fresh light feeling can be achieved without sticky feeling for the feeling of use, but also excellent transparency in its appearance can be achieved.

(C) An oil used in the present invention is any oil being semisolid or liquid at room temperature, which is usually used in cosmetics. The (C) oils include, for example, higher alcohols such as cetyl alcohol, isostearyl alcohol, lauryl alcohol, hexadecyl alcohol and octyldodecanol; fatty acids such as isostearic acid, undecylenic acid and oleic acid; polyhydric alcohols such as glycerin, sorbitol, ethylene glycol, propylene glycol, polyethylene glycol and raffinose; esters such as myristyl myristate, hexyl laurate, decyl oleate, isopropyl myristate, hexyldecyl dimethyloctanoate, glycerin monostearate, diethyl phthalate, ethyleneglycol monostearate and octyl oxystearate; hydrocarbons such as liquid paraffin, vaseline and squalane; waxes such as lanoline, reduced lanoline and carnauba wax; fats and oils such as mink oil, cacao oil, coconut oil, palm kernel oil, camellia oil, sesame oil, castor oil and olive oil; fluorinated oils such as perfluoropolyether; and ethylene/α-olefin/co-oligomer, etc.

Further, as (C) oil which is different from the oil mentioned above, there may also be used, for example, silicone compounds such as dimethylpolysiloxane, methylphenylpolysiloxane, polyether-modified organopolysiloxane, alkyl-modified organopolysiloxane, terminal-modified organopolysiloxane, polyglyceryl-modified silicone, amino-modified organopolysiloxane, silicone gel, acrylic silicone and trimethylsiloxy silicic acid.

In the present invention, more than one or two types of these (C) oils can be used in combination. There may be preferably mentioned, for example, a combination of hydrocarbons such as liquid paraffin and silicone compounds such as dimethylpolysiloxane and methylphenylpolysiloxane. Among these, there may be preferably used one in which refractive index of the oil content is preferably 1.4 to 1.6, more preferably 1.45 to 1.54. Further, when measuring the refractive index (n) in the present invention, 2g of a sample is first taken on a glass plate and the sample is kneaded using a cabinet scraper until it has a homogenous slurry shape, which is then measured at 20°C using Precision Abbe Refractometer (manufactured by Atago Co., Ltd., type: 3T).

Taking account of the usability as a powder cosmetic, the blending amount of the (C) oil is, based on the total amount of the cosmetics, 25 to 55% by mass, preferably 30 to 55%, more preferably 30 to 50%. If the blending amount is within such range, a property as a powder formulation is maintained while it has excellent transparency in its appearance and better feeling of use without sticky feeling.

In the solid powder cosmetics of the present invention, optionally, in addition to the (C) oil mentioned above, a volatile oil as (C) oil can be further blended in the blending amount of 3% by mass or less based on the total amount of the cosmetics, preferably 1% or less.

Here, as a volatile oil, there may be exemplified by isoparaffinic hydrocarbon having a boiling point within the range of 100 to 260°C; volatile silicone having a silicon number of 2 to 8; and methyl tristrimethyl siloxysilane. The isoparaffinic hydrocarbon having a boiling point within the range of 100 to 260°C includes a light liquid isoparaffin having a carbon number of 12 to 28 obtained by hydrogenating a copolymer of isobutene and n-butene, and a light liquid paraffin having a carbon number of 6 to 30 which is a volatile hydrocarbon mixture obtained by purifying petroleum, and, for example, NAS-3, NAS-4 and NAS-5 from NOF corporation; Isopar C, Isopar D, Isopar E, Isopar G, Isopar H, Isopar K, Isopar L and Isopar M from Exxon corporation; Shellsol 71 from Shell Oil Company; Nisseki Isosol 300 and Isosol 400 from Nippon Oil Corporation; Soltrol 100, Soltrol 130 and Soltrol 22 from Philips Petroleum Co.; and the like can be listed. The volatile silicone having a silicon number of 2 to 8 includes octamethylcyclotetrasiloxane, decamethylcyclopentasiloxane, tetradecamethylcyclohexasiloxane, octamethyltrisiloxane, decamethyltetrasiloxane, and the like. Examples of commercially available products of these volatile silicones include KF-96A-lcs, KF-96A-1.5cs and KF-995 from Shin-Etsu Chemical Co., Ltd.; SH244, SH344, SH245, DC345, DC246, SH200C-1cs and SH200C-1.5cs from Toray Dow Corning Co., Ltd.; TSF404, TSF405, TSF4045, TSF451-lA and TSF451-1.5A from GE Toshiba Silicone Co., Ltd.; and the like. Methyl tristrimethyl siloxysilane is also known as methyl trimethicone, and an example of a commercially available product thereof includes TMF-1.5 from Shin-Etsu Chemical Co., Ltd., and the like.

In the solid powder cosmetic of the present invention, in order to adjust the feeling of the cosmetic, powders having a refractive index within the range of 1.3 to 1.6 can be optionally further blended as an optional component. More preferably, powders having a refractive index within the range of 1.45 to 1.54 are selected. Specifically, among polystyrene resin, polyester resin (PET, etc.), polyamide resin (nylon, etc.), polyolefine resin (polyethylene, polypropylene, etc.), acrylic resin (polymethyl methacrylate (PMMA), etc.), polyurethane resin, silicic acid anhydride, water-containing silicic acid, polytetrafluoroethylene, and the like, powders having the above-mentioned refractive index can be selected. Further blending of the powders having a refractive index within such range allows adjustment of the feeling of the cosmetic, as well as allows to further improve the adhesiveness to a puff or a brush for powders (easiness to take) while maintaining transparency.

In the solid powder cosmetic of the present invention, in order to achieve more beautiful appearance, it is also possible to optionally further blend, as a optional component, a dye, a colored pigment, a white pigment, an organic powder, a photoluminescent powder, or the like, not to such an extent as to spoil the effect of the present invention. In particular, the blending of the photoluminescent powder causes no dullness to coloring and pearly feeling as compared with a common nontransparent powder cosmetic, so that the beauty of the appearance can be further emphasized.

Examples of dyes, pigments and organic powders used in the present invention include dyes and lake colors such as Red No. 104 Aluminum Lake, Red No. 102 Aluminum Lake, Red No. 226, Red No. 201, Red No. 202, Blue 1 Aluminum Lake, Yellow 4 Aluminum Lake, Yellow 5 Aluminum Lake and Yellow 203 Barium Lake; colored pigments such as yellow iron oxide, bengala, black iron oxide, chrome oxide, carbon black, ultra marine blue and iron blue; white pigments such as zinc oxide, titanium oxide, cerium oxide, particulate titanium oxide and particulate zinc oxide; extenders such as talc, mica, sericite, kaolin, plate barium sulfate and boron nitride; metallic salts such as barium sulfate, calcium carbonate, magnesium carbonate, aluminum silicate, magnesium silicate, magnesium myristate and zinc stearate; inorganic powders such as alumina; acrylic polymers such as methyl methacrylate; and organic powders such as polyethylene, nylon, polyurethane, vinyl chloride polymer, cellulose powder, chitin powder, chitosan powder and silk powder.

Further, examples of the photoluminescent powders include a pigment having photoluminescence generally used for cosmetics, such as titanium oxide coated mica(micatitanium), titanium oxide coated talc, titanium oxide coated synthetic phlogopite and titanium oxide coated glass powder. Among these, one or more types of powders can be appropriately selected and used. There is no particular limitation on the shape of these powders (such as spherical, rod-like, needle-like, plate-like, amorphous, scale-like and spindle-like). The powder is preferably one having a particle size within the range of 1 to 120 µm, more preferably within the range of 10 to 80 µm.

The blending amount of these dyes, colored pigments, white pigments, organic powders, photoluminescent powders, and the like varies depending on the use and is, without particular limitarion, 0.001 to 5% by mass based on the total amount of the cosmetics, preferably 0.01 to 1%. If the blending amount is within such range, the beauty of the appearance can be further emphasized while maintaining transparency.

Moreover, in the solid powder cosmetic of the present invention, aside from the above-mentioned powders, it is possible to optionally blend components which are generally added as a optional component, not to such an extent as to spoil the effect of the present invention. These components include bioactive components such as ultraviolet absorbers, preservatives, perfumes, antioxidants, moisturizer, vitamins, ceramides and plant extracts.

In the solid powder cosmetic of the present invention, the total blending amount of the above-mentioned essential components (A)-(C) is 70% by mass or more based on the total amount of the cosmetics, preferably 90% by mass or more, more preferably 95% by mass or more. The total blending amount of the essential components (A)-(C) is selected in the range of 100% by mass or less based on the total amount of the cosmetics. In addition to the essential components (A)-(C), the above-mentioned powders having a refractive index within the range of 1.3 to 1.6 can be blended in the range of 0 to 30% by mass based on the total amount of the cosmetics, preferably 0 to 10% by mass, more preferably 0 to 5% by mass. In addition and optionally, a dye, a colored pigment, a white pigment, an organic powder, a photoluminescent powder, and a component which is generally added, and the like can be blended.

In order to produce the solid powder cosmetic of the present invention, no particular device for production is needed; it is only necessary to follow a conventional production process used for the production of foundations and pressed powders. Any method can be adopted, for example, a method of filling the components (A)-(C) and optional any components into a container and, if caking, pressurizing and compressing powders on a metallic tray or a resin tray, and a method of mixing a mixture of component (A), component (B) and (C) oil before compression and optional any components with a solvent such as ethanol, filling it into a metallic tray, a resin tray or a container, and drying the solvent to cake. An example is as follows: components (A)-(B) mentioned above and optional any components are mixed homogenously, subsequently (C) an oil is gradually added thereto, which is mixed using a cutter mill, etc. until it has a more homogenous state, and the resulting mixed powder is filed into a metallic tray to have a desired thickness and then it is press-filled. The punching pressure can be set appropriately, for example, 2 kg/cm².

The transparent solid powder cosmetic of the present invention, as far as powders are caked as a formulation, can be made into any shape of cosmetics. The solid powder cosmetics filled in a container and caked include, for example, foundation, pressed powder, cheek color, face color, eye shadow, or the like. In addition, the solid powder cosmetics caked and molded from powders per se include powder stick or the like. In particular, the pressed powder is a cosmetic in which the effect of the present invention can be easily realized.

The solid powder cosmetics of the present invention obtained by blending the above-mentioned components and caking have transparency in its appearance and is excellent in its usability, as well as is excellent in its feeling of use such as dry feeling and fresh light feeling without sticky feeling during application; Further, the cosmetic having "transparency" referred to as in the present invention means the one in which, when a paper with a black character on it in a predetermined size written on a white background is placed on the bottom of the metallic tray, and onto the paper on the bottom a mixed composition is loaded to mold it to have a thickness of 3 mm, the character on the bottom can be visually recognized if seen from directly above.

### [Example]

Next, the present invention will be explained, referring to examples and comparative examples. Further, evaluation methods for various properties of cosmetics used in examples and comparative examples are shown below.

The evaluation criteria for "transparency" and "usability" used in the present invention are as follows:

(1) Evaluation of transparency
Evaluation of transparency was conducted, by observing a sample by visual inspection under the condition mentioned above, in a criteria below:
Case where the character (16 points) on the bottom of the metallic tray was recognized:⓪
Case where the character (26 points) on the bottom of the metallic tray was recognized:○
Case where the character on the bottom of the metallic tray was not recognized: X .

(2) Evaluation of usability
Only the samples of which the transparency of the appearance was evaluated as ⓪ or ○ in the (1) evaluation of transparency (other than Comparative Examples 4 and 5) were applied to 20 subjects using a brush, to conduct sensory evaluation. Score concerning "easiness to take" during use was set in 5 grades of "Good (5 points); Slightly good (4 points); Average (3 points); Slightly bad (2 points); Bad (1 point)" for evaluation, and score concerning "feeling" during use was set, with respect to "dry feeling" and "fresh light feeling", in 5 grades of "Feel (5 points); Slightly feel (4 points); Average (3 points); Not feel slightly (2 points); Not feel (1 point)" for evaluation. Further, average values of the scores of 20 subjects were obtained, so that the values were judged according to the evaluation criteria shown in Table 1.

**Table 1**

| Average Value of Scores | Judgment |
|---|---|
| 4.0 - 5.0 | ⓪ |
| 3.0 - 4.0 | ○ |
| 2.0 - 3.0 | Δ |
| 1.0 - 2.0 | × |

### Examples 1 to 8

Pressed powders were prepared according to formulations shown in Table 2 in examples concerning the cosmetic of the present invention, and were prepared according to formulations shown in Table 3 in comparative examples.

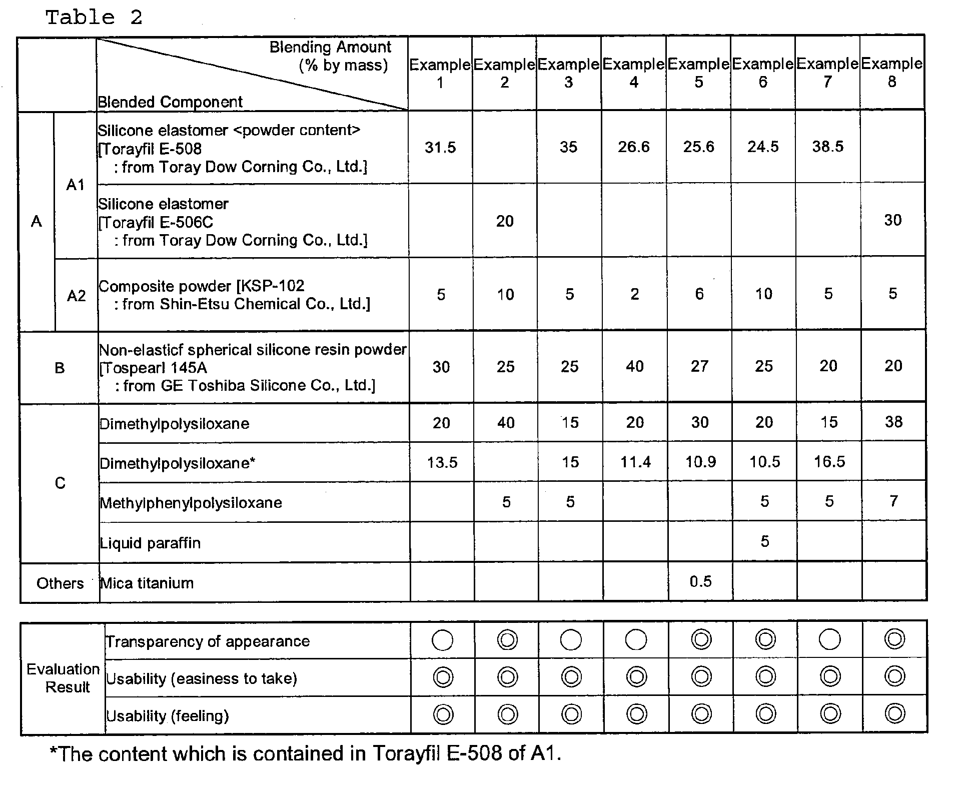

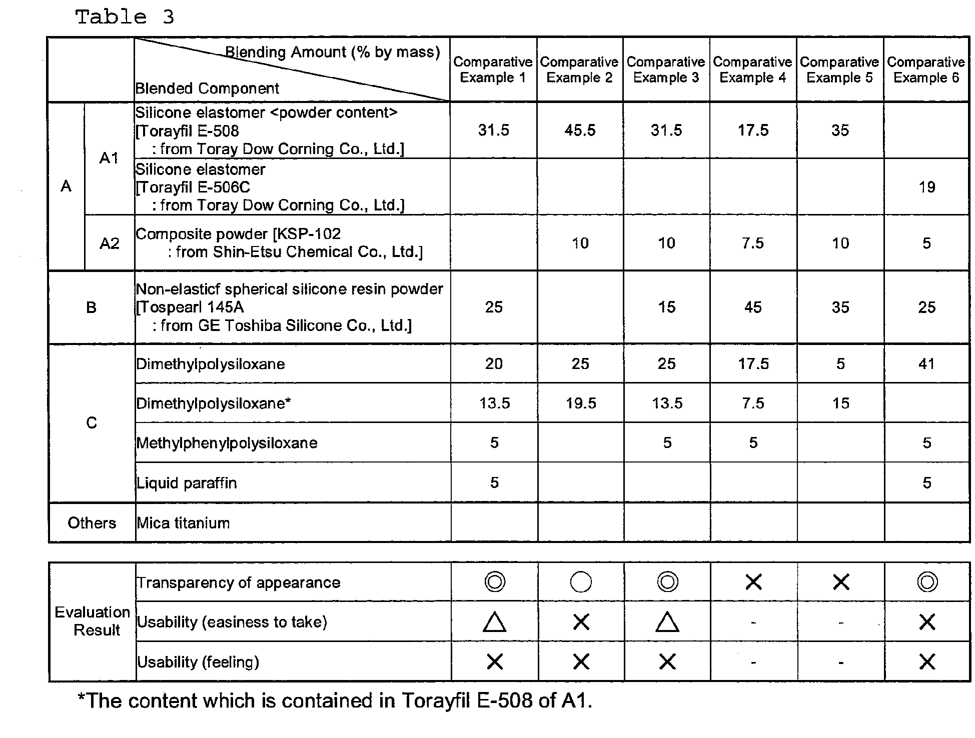

### [Production Process]

For producing the pressed powder for Examples and Comparative Examples, powder components are mixed homogenously, subsequently an oil component was gradually added thereto, and mixing was performed using a cutter mill until it had a more homogenous state. The resulting mixed powders were press-filled into a round metallic tray having a diameter of 55 mm and a height of 3 mm so as to have a sample thickness of 2 mm. The punching pressure is 2 kg/cm² and the amount of the content is about 3.0 to 3.5 g.
Incidentally, the average particle size of Torayfil E-508 used in Example and Comparative example mentioned above is 3 µm; the average particle size of Torayfil E-506C is 3 µm; the average particle size of KSP-102 is 30 µm; and the average particle size of Tospearl 130 is 3 µm.

From the results of the evaluation shown in Table 2, one can see that excellent evaluation results were obtained in the cosmetics of Examples 1-8 of the present invention for each item of "transparency of appearance" and "usability such as easiness to take and feeling." In contrast thereto, the results of the evaluation shown in Table 3 indicates that all the Comparative Examples in which transparency of appearance is evaluated as ⓪ or ○ of the cosmetics in Comparative Examples 1-6 results in causing sticky feeling in the evaluation item of feeling.

### Example 9

A pressed powder having a composition indicated below was prepared according to a conventional method. When it is evaluated for above-mentioned transparency of appearance and usability (easiness to take and feeling) by the same manner as that of above-mentioned, it was confirmed that the pressed powder has excellent transparency and also good usability.

| (Pressed powder) | (% by mass) |
|---|---|
| Silicone elastomer (powder content) [Torayfil E-508: from Toray Dow Corning Co., Ltd.] | 26 |
| Composite powder [KSP-102: from Shin-Etsu Chemical Co., Ltd.] | 6 |
| Non-elastic spherical silicone resin powder [Tospearl 145A: from GE Toshiba Silicone Co., Ltd.] | 26 |
| Dimethylpolysiloxane | 30 |
| Dimethylpolysiloxane (contained in Torayfil A) | 11 |
| Silicic acid anhydride | 1 |
| | 100 |

### Example 10

A pressed powder having a composition indicated below was prepared according to a conventional method. When it is evaluated for above-mentioned transparency of appearance and usability (easiness to take and feeling) by the same manner as that of above-mentioned, it was confirmed that the pressed powder has excellent transparency and also good usability.

| (Pressed powder) | (% by mass) |
|---|---|
| Silicone elastomer (powder content) [Torayfil E-508: from Toray Dow Corning Co., Ltd.] | 26 |
| Composite powder [KSP-102: from Shin-Etsu Chemical Co., Ltd.] | 6 |
| Non-elastic spherical silicone resin powder [Tospearl 145A: from GE Toshiba Silicone Co., Ltd.] | 26 |
| Dimethylpolysiloxane | 30 |
| Dimethylpolysiloxane (contained in Torayfil A) | 11 |
| Acrylate crosspolymer | 1 |
| | 100 |

### [Industrial applicability]

The transparent solid powder cosmetic of the present invention has novelty of being transparent in its appearance while being powder formulation, and is excellent in its usability such as easiness to take when using a puff, a brush or the like and in feeling such as dry feeling and fresh light feeling. Therefore, an unconventional and totally new solid powder cosmetic can be provided according to the present invention.

## Claims

1. A solid powder cosmetic comprising: (A) 25 to 55% by mass of elastic powder mixture; (B) 20 to 40o by mass of non-elastic spherical silicone resin powder with an average particle diameter within the range of 0.1 to 50 µm; and (C) 25 to 55% by mass of oil, as essential components, wherein (A) the elastic powder mixture comprises one or more types of each (A1) an elastic powder and (A2) a composite powder obtained by coating the periphery of an elastic powder with a non-elastic material, said solid powder cosmetic being obtained by caking a mixed composition of these essential components.

2. The solid powder cosmetic according to Claim 1, wherein the component (A1) is a silicone elastomer powder and the component (A2) is a composite silicone powder obtained by coating the surface of a silicone elastomer powder with a silicone resin.

3. The solid powder cosmetic according to Claim 1 or 2, wherein the component (B) is a silicone resin powder selected from methylsiloxane network polymer or polyorganosilsesquioxane hardened material.

4. The solid powder cosmetic according to any one of Claims 1 to 3, wherein the component (C) is a silicone compound and/or a hydrocarbon.

5. The solid powder cosmetic according to any one of Claims 1 to 4, further comprising powders having a refractive index within the range of 1.3 to 1.6.

6. The solid powder cosmetic according to any one of Claims 1 to 5, further comprising a photoluminescent powder.

7. The solid powder cosmetic according to any one of Claims 1 to 6, wherein the total blending amount of components (A), (B) and (C) is 90o by mass or more based on the total amount of the cosmetic.
